# EUROPEAN PATENT APPLICATION

(11) **EP 2 727 591 A2**
(43) Date of publication of application: **07.05.2014**
(21) Application number: 13188411.6
(22) Date of filing: 14.10.2013
(51) Int. Cl.: A61K 31/43, A61K 31/545

(54) **Stable pharmaceutical compositions containing cefaclor and clavulanic acid**

(30) Priority: 31.10.2012 TR 201212497
(71) Applicant: Kücükgüzel, Sükriye Güniz, Istanbul (TR)
(72) Inventor: Küçükgüzel, Sükriye Güniz, Istanbul (TR); Salli, Duygu, Istanbul (TR)
(74) Representative: Mutlu, Aydin

(57) **Abstract**

The present invention is related to pharmaceutical compositions that have been developed in order to increase the effectiveness spectrum of Cefaclor by means of the beta lactamase enzyme inhibitor characteristic of clavulanic acid.

## Description

### Technical Field

The present invention is related to pharmaceutical compositions that have been developed in order to increase the effectiveness spectrum of Cefaclor by means of the beta lactamase enzyme inhibitor characteristic of clavulanic acid. In this study, a combined suspension product study has been carried out between Clavulanic acid which is a ß lactamase enzyme inhibitor and Cefaclor which is one of the ß lactame group antibiotics that does not have medicine interaction and an analytic method has been developed.

### Prior Art (Known State of the Art)

Cefaclor (3-chloro-7-D-2-amino-2-phenylacetamido)-8-oxo-5-thia-1-azabicyclo[4.2.0]oct-2-en-2-carboxylic acid) (Formula I) is an active ingredient of the cell wall synthesis inhibitor cephalosporin group. It is administered orally. As Cefaclor was marketed after first generation medicines, it has been thought by clinicians that cefaclor was a second generation drug. However the effective spectrum represents first generation cephalosporins rather than second generation.

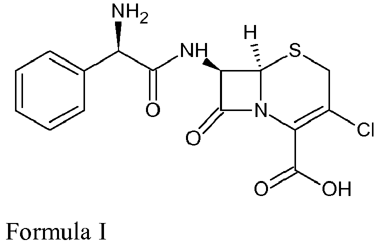

Cefaclor is actually effective as a bactericide. The bacteria primarily and specifically binds with the penicillin binder proteins (PBP's) that are located at the inner section of the bacteria cell wall and inhibits the third and last steps of the cell wall synthesis. The PBP's are responsible of a few steps of the cell wall synthesis and their numbers change between a few hundred to a few hundred thousand in each bacteria cell. For this reason, like other cephalosporins and penicillins, the activity of cefaclor against an organism also depends on binding to the necessary PBP in order to form a bactericidal effect located there and for it to be able to penetrate the bacteria cell wall. The prevention of cell wall synthesis related to PBP by Cefaclor in the end will lead to the lysis of the cell. The autolysins located on the bacteria cell wall will act as mediators to the lysis incident. The relationship between PBP's and autloysins are not known yet but it is possible for Cefaclor which is a beta lactame antibiotic to be in interaction with the autolysin inhibitor.

As a rule first generation cephalosporins are more effective than second and third generation cephalosporins against gram positive organisms. However their activities against gram negative bacteria is weaker. The gram positive organisms which it is effective on comprise *Staphylococcus aureus* (the strains that are resistant to meticilin are resistant) which produce penicillinase or which do not produce penicillinase and streptococcus (besides enterococcus). The gram negative organisms they are limitedly effective on comprise *Escherichia coli, Klebsiella specie and Pseudomonas mirabilis.* Approximately 10 - 15% of the ampicillin resistant *Haemophilus influenzae* is resistant to cefaclor. Not all of cephalosporins are effective against enterococcus. As Cefaclor is sensitive to beta lactamase hydrolisis, it is not effective agains some *Enterobacteriaceae* and *Moraxella (Branhamella) catarrhalis* which carries out beta lactamase.
Cefaclor which is a wide spectrum semi synthetic antibiotic, is a second generation cephalosporin used orally. It shows its bactericidal effect by damaging cell wall synthesis. Cefaclor, is effective against many bacteria which also comprise *H*. *Influenzae* and which is resistant against first generation cephalosporins.
The bacteria which are known to be sensitive to cefaclor are as followed: *Staphylococcus* (including types that carry out coagulase positive, coagulase negative and penicillinase), *Streptococcus pyogenes* (A group β-haemolytic streptococcus), *Streptococcus pneumoniae, Branhamella catarrhalis, H. influenzae* (including those species that carry out β-lactamase and those that are resistant to ampicillin), *E. coli, P. mirabilis, Klebsiella* species, *Citrobacter diversus, Neisseria gonorrhoeae, Propionibacterium acnes, Bacteroides* species (except *Bacteroides fragilis*)*,* peptococcus, peptostreptococcus.

Cefaclor is administered orally. It is resistant to acid and it is quickly absorbed from the gastrointestinal channel. When cefaclor was given orally the peak serum levels are reached in 30 - 60 minutes.

When taken together with food the serum peak concentration slowly decreases and the time to reach peak concentration is lengthened; but there will be no change in the total amount of the drug absorbed. Approximately 25 % of the drug in circulation is bound to plasma proteins. Cefaclor is distributed to many of the body fluids, however it cannot reach therapeutic levels in the cerebrospinal fluid. It effectively passes beyond placenta. Cefaclor is eliminated in urine with glomerular filtration and tubular secretion without significantly changing. It may pass on to breast milk at a low level. The elimination half life in patients with normal renal functions is 30 - 60 minutes. The patients who have severe kidney disease elimination half life can extend to 3 - 5.5 hours. However in such patients dosage adjustment is not necessary. Cefaclor is eliminated with haemodialisis. Cefaclor which is an acid resistant composition, is absorbed from the digestive tract. 250 mg (50 0mg) Cefaclor taken on an empty stomach will reach to 5 - 7 µg/mL (13 - 15 µg/mL) which is its highest serum concentration in 30 - 60 minutes. When taken after a meal, even if the total absorbed amount does not change, the highest serum concentration is low and it is reached later. Approximately 65 - 80 % of the dose taken, is eliminated through urine without changing in 8 hours. The excretion speed is relatively high in the first 2 hours. When taken at a dose of 250 mg (500 mg) in the first 8 hours a 600 µg/mL (900 µg/mL) Cefaclor concentration is obtained in urine. Serum half life is 0.6 - 0.9 hours. Although half life is extended in people with renal disorders, it is usually not necessary to carry out a dose adjustment.
Clavulanic acid ((*Z*)-(*2R*,*5R*)-3-(2-hydroxyethylene)-7-oxo-4-oxa-1-azabicyclo[3.2.0]heptane-2-carboxylic acid) (Formula II), is an antibiotic which is a beta lactamase inhibitor which is a clavam species obtained from *Streptomyces clavuligaris.* It is used as a potassium clavulanate. It is used in order to strengthen antibacterial by inhibiting beta lactamase enzymes in bacteria and in order to widen their effect spectrum.

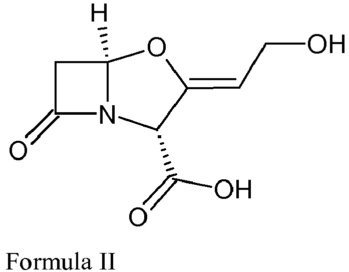

Clavulanic acid is a beta lactamase inhibitor; it inhibits beta lactamase irrevocably. It does not have much effect on the penicillin binding proteins (PBP's) which are located in the bacteria periphery. Other beta lactamase inhibitor drugs such as Clavulanic Acid, Sulbactam and Tazobactam are named as "suicide inhibitors". The reason for this is that they are broken down to small molecules by beta lactamase in the beginning and one of these broken pieces then inhibits the beta lactamase enzyme. Thus the enzyme is inactivated irrevocably by an agent that manifests from a piece that it has itself broken down. Clavulanic Asit principally inhibits Richmond-Sykes class II beta lactamase and class III and IV beta lactamase; it is ineffective against class I or namely to cephalospoinase.
As clavulanic acid does not touch PBP's generally like sulbactam, it does not show a significant effect on its own. As Staphylococcus which is resistant to ampicillin and amoxicillin produce beta lactamase, show synergic interaction with these two antibiotics against *M. catarrhalis, Neisseria gonorrhoeae, H. influenzae, Enterobacteriaceae* species (*E. coli, indol-positive Proteus, Klebsiella* etc) and *Bacteroides fraglilis.* The result is that all of these bacteria which are resistant are made to be sensitive against ampicillin or amoxicillin. A pharmaceutical suspension is a rough dispersion established with the distribution in a liquid medium, of a solid agent which has been turned into a fine powder which does not dissolve. The particle size in nearly all pharmaceutical suspensions is larger than 0.1 µm. In suspensions which have heterogeneous dispersion systems which look homogenous, the dispersed phase particles which have been distributed within a dispersion medium, precipitate in time. However it is desired for the pharmaceutical suspension's precipitation to be reversible, for it to disperse homogenously when shaken and that it is formulized such that it enables for a full dose of drug to be received each time.

Suspension formulations may comprise, an active ingredient, a wetting agent, a preserver colloid, flocculation, deflocculation and suspension agents, and a thickening agent as an inner phase; and as an external phase formulations may comprise a carrier (vehicle), pH adjusters (tampons), preservatives, anti foaming agent, colorant, fragrance and flavouring. Good formulated suspension must have some basic characteristics. The particle size distribution must be in a tight range and when precipitated they must not form a cake. The viscosity should be such that it is not too fluidy. It should be stable chemically, resistent to microbial contamination and at a homogenous appearance. The suspension drug shape is prepared in a small amount, and initially the coarse particled powder (d > 5mm) agents are ground in a mortar until a fine particle power (d = 1 - 5 mm) is obtained. The powder is added to the powder using the method of geometric decimation. A carrier (water) is added to the mixture in the mortar. The materials that are dissolvable in water are dissolved in the remaining amount of water and are added to the mortar and the obtained mixture is taken into a graduated cylinder. The remaining amount in the mortar is added into the graduated cylinder together with water and the desired volume is completed with this remaining amount and is filled into the packing container and labeled.

In addition a label that warns "shake before use" must be added.
Pharmaceutical suspensions can be classified as
- Orally applied mixtures
- Externally applied lotions and
- Suspensions (parenteral) for injection.
There are some important parameters which determine the quality, acceptability and performance of the suspension drug types. Some of the basic parameters that need to be taken into consideration when evaluating suspensions are as follows:
- appearance, scent, colour,
- pH
- intensity
- viscosity
- easy mixing
- particle size
- microbial content
- usage safety in humans
Chromotagraphy is the general name which encompasses many methods and which ensures that agents which are similar to each other both physically and chemically can be separated from each other, can be defined and determined. A mixture containing hundreds of materials can be analysed using the chromatography method.
This is a common analytical method that is used in order to separate, define and determine chemical components. Commonly there is a stable phase and a mobile phase in all chromatographic methods. The components within a mixture together with flowing gas and a liquid phase, are pass over a stable phase, separation is carried out depending on the migration speed of the components.
Chromatographic methods can be classified in two ways. When based on how the mobile and stable phasesphysically contact each other,the method will be separated into two as column chromatography and linear chromatography. The stable phase will be kept in a thin column in column chromatography and the mobile phase is forced to pass trhough this stable phase under pressure. In linear chromatography however, the stable phase is attached on a flat plate and the pores between paper and in this case the mobile phase moves with the effect of capillary or gravity between the stable phase. A sample in chromatographic separation, is carried in the system with a mobile phase which is a gas, liquid, or a super critical fluid. The mobile phase is passed through a stable phase which has been fixed at a solid surface or a colum and which does not mix with the mobile phase. The selection of these two phases targets the distribution at different rates of sample components in mobile and stable phases.

### High pressure Liquid Chromatography

High pressure Liquid Chromatography (HPLC) is based on the separation of the compounds in a mobile and stable phase. The separation is carried out by dispersion in accordance with the type of phase, adsorption or by an ion exchange procedure. This method is used commonly in determining the amount of drug active ingredients in pharmaceutical preparations and body fluids. Th,s is a method which is commonly used and which is mentioned in the pharmacopea as it yields a precise result especially in preparates which have low active ingredients, as the analysis can be completed in short period of time and as the mistakes that could be made by the people carrying out the analysis can be minimized.
With the advanced technology in the recent years, a particle dimension of 3 - 10 µm and approximately 5mm diametered columns have been developed. These developments have ensured that chromatographic separation is increased,the performance of chromotagraphic systems have increased and the analysis times have shortened.
High pressure Liquid Chromatography has some advantages in comparison to other chromotagraphic methods.
i. The column separation peformance is high
ii. The procedure is less dependent on the user and for this reason it can be repeated.
iii. It is suitable for quantittive analysis.
iv. The analysis time is shorter in comparison to other methods.
v. It is flexible enough to provide operation in wide concentration ranges.
vi. It is applied in low temperatures
vii. It is possible to be able to choose a detector in accordance with the characteristics of the material to be analyzed.
With high pressure liquid chromatography, aminoacids, nucleic acids, medicine, carbohydrates, proteins, hydrocarbons and similar material can be analyzed.
1. Mobile phase chamber,
2. Coupling equipments and mobile phase application system,
3. Mobile phase pump
4. Automatic sampler,
5. Column
6. Detector,
7. Recorder

### Developing an analytic method

The first step when developing an analytical method is to research literature which comprises methods wherein the same or similar equipments are used. The development of a new or improved method is adapted generally to equipment, the analyte to be worked on, targets and the requirements of the method. It is necessary to choose the requirements of the method, and to determine why a certain type of equipment needs to be selected. Moreover the analytic column that needs to be worked on, the mobile phase, detector and the amount determination method are points that need to be decided on during the method development stage.
There are several valid reasons to develop a new analysis method. The anticipated results may not be obtained allways through the present methods or approaches based on literature. The methods may be faulty or their reliability may be low.
There may be methods which are expensive,which require a lot of time and energy, which are unsuitable for routine usage and which do not have sufficient selectivity or sensitivity regarding samples. When it is thought that the correctness and precision of analytic data could be increased with new equipments and techniques, it is deemed always necessary to develop methods which are sensitive, susceptive, precise, repeatable, economic and easy to apply and unproblematic.

When optimizing a method, values such as, the peaks obtained, the tailing factor, resolution, assymetry, capacity factor, and retetion time are taken into consideration. The symettry must reach the highest point possible, the quantitative determination limit must be determined at the lowest point, the retention time must be short, it must be linear at a wide range, recovery and precision must be provided.

### The validity of the analytic method

The analytic methods used in the health sector, will help in biological sample analysis, the evaluation and interpretation of pharmaco kinetic data, in qualitative and quantitative analysis of drug active agents, and determination of the impurities and metabolytes of drug active materials.
In order to be able to carry out these procedures and in order to obtain reliable results completely valid analytic methods need to be used. Validity according to Green's definition are proceures that enable the analytic method to be suitable for the desired aim.
Validity is defined to be, all of the procedures carried out that show the performance of the device, method or system. The success principles of a method is determined according to the aim of the method that is to be validated and the scope of the method. The validity indications of a method, are precision, correctness, limit determination, limit of quantification, selectivity, specificity, linearity, measurement range, susceptibility and stability. The validation of a method, must be carried out when a method is being carried out for the first time in a laboratory, when developing a new method for analysis, making a change in the method that is being used, when a validated method is going to be used in a different laboratory, if there is going to be a change in the device that is to be used or when the user in charge is going to change, or if a change in time has been observed regarding the success rate of the method, according to the quality control tests.
According to the American pharmacopea a validation indicates the linearity, correctness, precision, repeatability and reliability, at a chosen range, of an analysis method that has been developed for the material to be analyzed.
Method validation is carried out when;
- Any kind of method is going to be carried out in a laboratory for the first time.
- A new method is developed for an analysis
- A change is carried out reagrding a method that is being used
- A validated method is going to be carried out in another laboratory
- It is thought that there is a change in the performance of the method during quality control testing.

### General procedures of validation

1. Controlling and validating a device (applicable for all harware and softwares)
2. Validation carried out in order to prove the validity of a method that has been developed or after the changes that have been made in the parameters of a method that has been developed.
3. System compatibility tests (SUT) carried out for separation methods after the device and the related method has been selected an their validations have been carried out. These tests are tests that have been recommended by the FDA and USP and these are tests which for a part of developing a method for liquid chromotagprahy and other separation techniques.

### Typical validation parameters

### Selectivity

Selectivity can be defined as the competence in providing separation and determination of an analyte in the presence of other components in a sample in the analytic method and to be able to asnwer directly for a single analyte.

### Correctness

It is defined as the proximity of the compatibility between the reference value which is accepted to be correct in an analytic method and the value found. The results are given as an approximate recovery %. It shows the rate of recovery of the agent from the medium where an analysis is carried out when an agent is added to the analysis method. At least five measurements need to be taken in three concentrations within a given range. This is carried out for at least 3 different concentrations being low, medium and high. The medium concentration analysis must be the same with the sample concentration carried out. The correctness must not show a deviation higher than 20 % for a measurement base limit. The approximate value must be within the range of ± % 15 of the real value. The deviation rate of the approximate value from the real value is accepted to be the correct measurement.

### Precision and repeatability

The precision is given with the proximity and relative standard deviation (%) of the values obtained as a result of a test. Repeatability defines the compatibility of results obtained from samples which have been prepared with the same method under different conditions. The precision study results can be used for repetition.

### Calibration curve and linearity

This is the relationship between the response of the detector and the analyte concentrations.It must be formed for each analyte in the sample and a sufficient amount of standards should be used. At least 5 inner limit concentrations must be present in a calibration curve.
Linearity is to have the point at a linear line on the graphic drawn and for the answer against concentration to be changed linearly. Correlation coefficients (r) are parameters which give linearity. The regression analysis against the measured values of the analyte concentration is calculated mathematically. If the correlation coefficent is r = 0,999 or higher and if the answer values are above the drawn line then linearity is acheived. In order to determine linearity, at least 5 different concentrations need to be prepared and analyzed from the stock solutim. The concentration has to be chosen at such a range that the bottom limit is 50% lower than the target lecel and the top limit is 150% higher than the target level. The procedure to determine the linearity carried out with these 5 values need to be repeated at least 3 times. For the complete validation of linearity, also the BSS % of the curve and intersection values or standard errors must be completed and must be given in the report.

### Study range

The study range is the concentration range between the bottom and top limits where the method having sufficient linearity, correctness and susceptibility, is valid. The concentration range generally changes according to the chosen method type. This range is determined after the linearity of the method is found. The concentrations of standards in analyzing the primary material is usualy measured at the targeted level or close to said targeted level. The bottom limit of this concentration must be chosen at a range which is 50 % lower and 150 % higher than the target level. The range can be arranged such that it is 50, 75, 100, 125 and 150 % of the target level.

### Stability

Stability is the measurement which shows how much and how the analysis results are affected when the method parameters are changed. Stability makes it easier to decide if the method needs re-validation when one or more indications of the method are changed. The ICH documents recommend that the stability parameters need to be evaluated when a method is going to be developed.

### Description of the Invention

The present invention is related pharmaceutical compositions developed in order to increase the effect spectrum of cefaclor by means of the beta lactamase enzyme inhibitor characteristic to clavulanic acid.

In this study, a combined suspension product (CLACEF) study has been carried out between clavulanic acid which is a ß lactamase enzyme inhibitor and Cefaclor which is one of the ß lactamase group antibiotics which do not have medicine interaction and an analytic method has been developed.
Materials such as sodium pentane sulfonate, methanol, ortho phosphoric acid, triethylamine chemical materials, aerosol, sodium saccharine, Avicel pH 1.2, xanthan gum, sodium benzoate and strawberry flavour have been used in this study.

### Column

Inertsil ODS 3V type column has been used (Column characteristics: 250 mm, 4.6 mm, 5 µm)

### Devices/Equipment Used

High pressure liquid chromatography (Agilent 1200)
pH metre (Sartorious Professional pH Meter PP-20)
Intensity device (REM Kyoto Electronics Model DA- 130N)
Magnetic mixer-heater (Heldolph MR 3001)
Scale (Mettler Toledo)
Ultrasonic Bath

### Preparation of Solvents

### Mobile Phase

Sodium pentane sulfonate salt (1.0 g) is dissolved with 780 mL water. Triethylamine (10 mL) and methanol (220 mL) is added and mixed. Following this pH is adjusted to approximately 2.5 by ortho phosphoric acid. It is drained out of a membrane filtre (0.45 µm) and is kept in an ultrasonic bath for 15 minutes thus eliminating its gas.

### Solvent

Mobile phase is used as a solvent.

### Standard Solution

Precisely weighed 25.0 mg Cefaclor and 12.0 mg potassium clavulanat is taken into a 20.0 mL volumetric flask. An amount of solvent is added and it is kept in an ultrasonic bath for 3 minutes so that it is dissolved. The volume is completed with a solvent. PVDF is drained from a filter (0.45 µm) and is bottled. (C_{Cefaclor}: 1.25 mg/mL, C_{Potasyum Klavulanat}: 0.6 mg/mL)

### Test Solution

Precisely weighed 5.0 g suspension sample is taken into a 200.0 mL volumetric flask.
An amount of solvent is added and it is kept in an ultrasonic bath for 5 minutes so that it is dissolved. The volume is completed with a solvent. 5.0 mL of this solvent is taken into a 100 mL volumetric flask. The volume is completed with a solvent. PVDF is drained from a filter (0.45 µm) and is bottled. (C_{Cefaclor} : 1.25 mg/mL, C_{Potasyum Klavulanat}: 0.6 mg/mL)

### High pressure liquid chromatography studies

### How the test is carried out

In order to clean the lines and to eliminate the air that is trapped inside the channels before injecting Cefaclor and clavulanic acid into the high pressure liquid chromatography (YPSK) system, it is prevented for the flow to pass through the column and deionized water is passed through the lines with a spead of 5 mL/minute flow speed. The column is connected after the column compartment is brought to a suitable temperature. Following this mobilephase is passed through the column. It is enabled for the column to be saturated and for the pressure to be fixed. By this means the system is made to be suitable for analysis. When the system is being closed the column is cleaned with a 5 % methanol water and methanol with a gradient system. Lukewarm deionized water is passed through the lines and cleaned.

### Developing the method to determine an amount of Cefaclor and Clavulanic acid with YBSK

With the method that is to be developed it has been aimed to ideally separate Cefaclor and Clavulanic acid with reverse phase liquid chromatography.
The chromatographic parameters that need to be taken into account in order to determin the most suitable chromatographic conditions for simultaneaously analysing Cefaclor and Clavulanic acid with high pressure liquid chromatography are retention time, capacity factor, theoretical layer number and peak symmetry rate.
Attention was paid such that the retention times of the peaks arriving from placebo and the solvent were the same with the retention times of the active ingredients.

### Optimization of the high pressure liquid chromatography System

A method has been developed by using the Cefaclor USP (American pharmacopea) amount determination method. The method is carried out with high pressure liquid chromatography.
Column: Inertsil ODS 3 V 250 mm, 4.6 mm, 5 µm
Chromatographic conditions:
   Flow veolcity: 1.5 mL/minute
   Detector: 265 nm
   Injection volume: 20 µL
   Analysis period: 25 minutes (USP 34)

The parameters such as mobile phase organic adjuster rate, mobile phase pH, wave lenght, and flow velocity have been changed in the separation of cefaclor and clavulanic acid with reverse phase liquid chromatography and its effects have been examined.

### Cefaclor and Clavulanic acid suspension (CLACEF) formulation examples

### Example 1

The formulation comprises 5000 mg cefaclor (5334.4 mg with potency), 500 mg aerosil, 200,250 mg aspartame, 1670 mg Avicel pH 102 each having been sieved from a 500 µm sieve (The formulation must comprise a total of 3040 mg Avicel). As the used active ingredient is Potassium Clavulanate: Avicel (1:1), 1670 mg Avicel is added; and thus the pre-determined amount of Avicel has been added. 100 mg Xanthan gum, 140 mg Sodium Benzoate, 30 mg Succinic acid, 300 mg Vanilla essence have all been weighed with precision scales, and are mixed inside a vessel for 5 minutes such that the mixture is homogenous. Following this 1140 mg Clavulanic acid: Avicel (1:1) (2748.31 with potency) is weighed precisely and added to the same vessel. Care is taken to ensure the mixture is homogenous and the powder is shaken for 5 minutes. The prepared powder is diluted with de-ionized water and is left to swell for some time; following this it is topped off with 100 mL de-ionized water. It has been observed that there was no swelling in the suspension. The intesity was low. It has been determined that the suspension had an intensity of 0.991 g/cm³ when measured. The scent and the taste of the suspension was not as desired. In the amount determination study carried out, recovery was provided with clavulanic acid whereas with Cefaclor the % of recover was recorded to be low. It has been determined that with this formulation a complete dissolving was not obatined when cefaclor was used and it has been decided that the study was not suitable to the Cefaclor 250 mg/5 mL dose.

### Example 2

The formulation comprises 2500 mg cefaclor (2667.244 mg with potency), 500 mg aerosil, 200,250 mg sodium saccharin, 1670mg Avicel pH 102 each having been sieved from a 500 µm sieve (The formulation must comprise a total of 3040 mg Avicel). As the used active ingredient is Potassium Clavulanate: Avicel (1:1), 1670 mg Avicel is added; and thus the pre-determined amount of Avicel has been added. 200 mg Xanthan gum, 140 mg Sodium Benzoate, 30 mg Succinic acid, 300 mg strawberry essence have all been weighed with precision scales, and are mixed inside a vessel for 5 minutes such that the mixture is homogenous.
Following this 1140 mg Clavulanic acid: Avicel (1:1) (2748.31 with potency) is weighed precisely and added to the same vessel. Care is taken to ensure the mixture is homogenous and the powder is shaken for 5 minutes. Sodium saccharin was used instead of aspartame as it has a sweetening characteristic which is 300 times more than saccharose. Thus it was aimed to ensure that the taste was better. The Xanthan gum amount was increased two time the amount more. As a result the suspension swelled more and suspended better. The amount of Cefaclor was increased by 50 %. By this means the dissolving problem of Cefaclor is eliminated and the recorvery of Cefaclor was close to 100 % with the suspension prepared at a dose of 125/5 mL. As a result a 100 mL suspension powder was prepared which comprises 125 mgCefaclor and 57 mg Clavulanic acid in each 5 mL.

### Cefaclor and Clavulanic acid wavelength screening

After Cefaclor and Clavulanic acid is dissolved with the solvent mentioned in the method, a wavelength screening has been carried out within high pressure liquid chromatography having a DAD detector. A wavelength where the absorbance is not effected much from the small changes in the wave lenght value needs to be chosen. This will enable for the measurements to be carried out within the maximum or minimum wavelength values within the absorption spectrum. The wave length where cefaclor performs the most absorbance is 264 nm; the wave length where clavulanic acid performs maximum absorbance is 210nm. When we look at the spectrums during the screening, it can be observed that a wave length which is common for the maximum absorbances cannot be chosen. For this aim, the wave length has been determined as 238 nm as the mimimum absorbance wave lengh of cefaclor. As a result at least a stable wave length was determined for Cefaclor.

### Description of the Drawings

**Figure 1****:** UV/GB spectrum of the solution prepared with Cefaclor 1.25 mg/mL Concentration.
**Figure 2****:** UV/GB spectrum of the solution prepared with a 0.6 mg/mL concentration of Clavulanic Acid.
**Figure 3****:** Cefaclor Clavulanic Acid Standard Chromatogram carried out with pH 3.5 mobile phase.
**Figure 4****:** Sample Chromatogram carried out in the analysis with pH 3.5 mobile phase
**Figure 5****:** Cefaclor Clavulanic Asit 100 % Standard Chromatogram
**Figure 6****:** Cefaclor Clavulanic Acid sample Chromatogram
**Figure 7****:** Chromatogram 0.6 mg/mL Clavulanic acid 1.25mg/mL Cefaclor where the system compatibility parameters are calculated.
**Figure 8****:** Cefaclor System compatibility parameters
**Figure 9****:** Clavulanic Acid System compatibility parameters

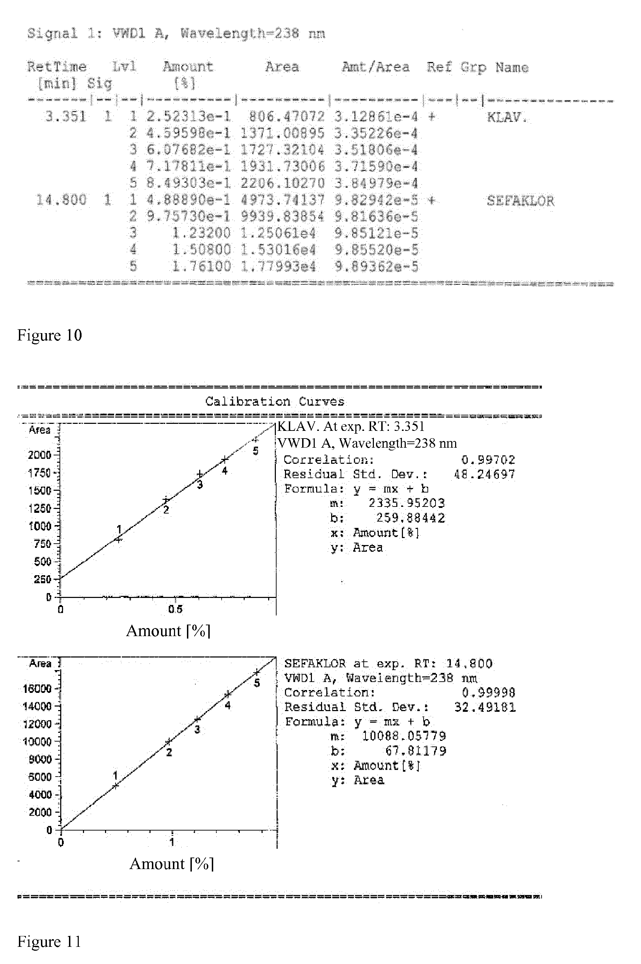

**Figure 10****:** Cefaclor Clavulanic Acid calibration table
**Figure 11****:** Cefaclor Clavulanic Acid calibration lines
**Figure 12****:** Cefaclor Clavulanic Acid Standard Chromatogram (100 % standard) 0.6 mg/mL Clavulanic Acid, 1.25 mg/mL Cefaclor
**Figure 13****:** 0.6 mg/mL Clavulanic Acid 1.25mg/mL Cefaclor precision sample Numunesi-1 Chromatogram calculated by device for Clavulanic Acid

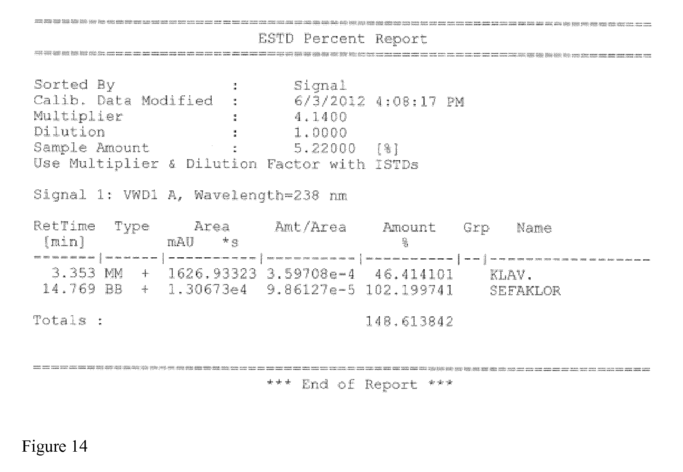

**Figure 14****:** Clavulanic Acid 1.25 mg/mL Cefaclor Precision sample-1 Chromatogram calculated by device for cefaclor.

### Determining flow velocity

In order to decrease the retention time of the peaks, the flow velocity has been carried out in 2.0 ml/minute. Other conditions were left the same. It has been seen that clavulanic acid peaks combined with solvents and placebo peaks and that there was not a good separation. For this reason it has been allowed for the flow velocity to remain at 1.5 mL/minute.

### Determining the mobile phase

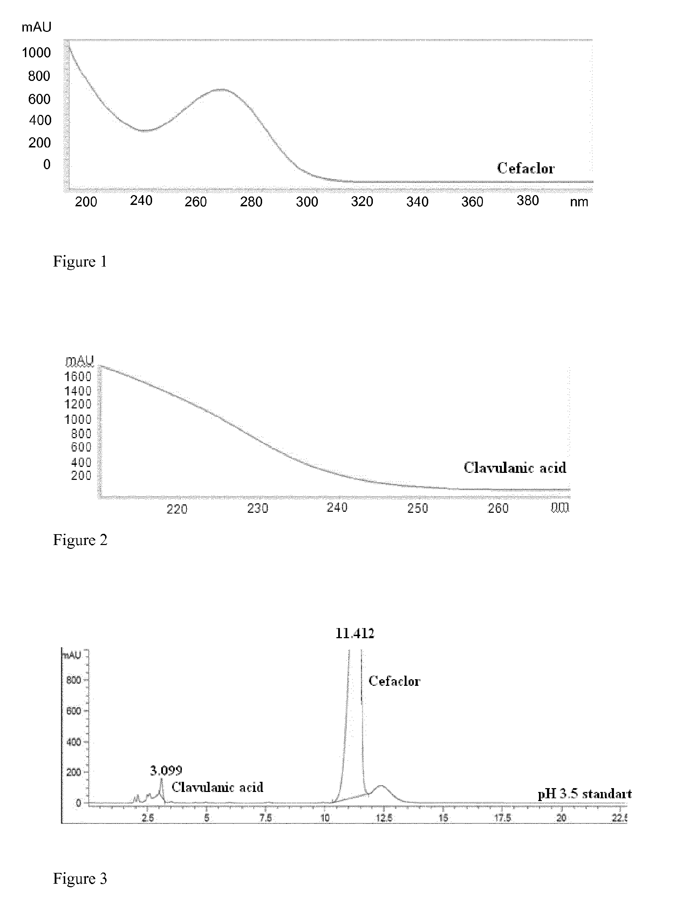

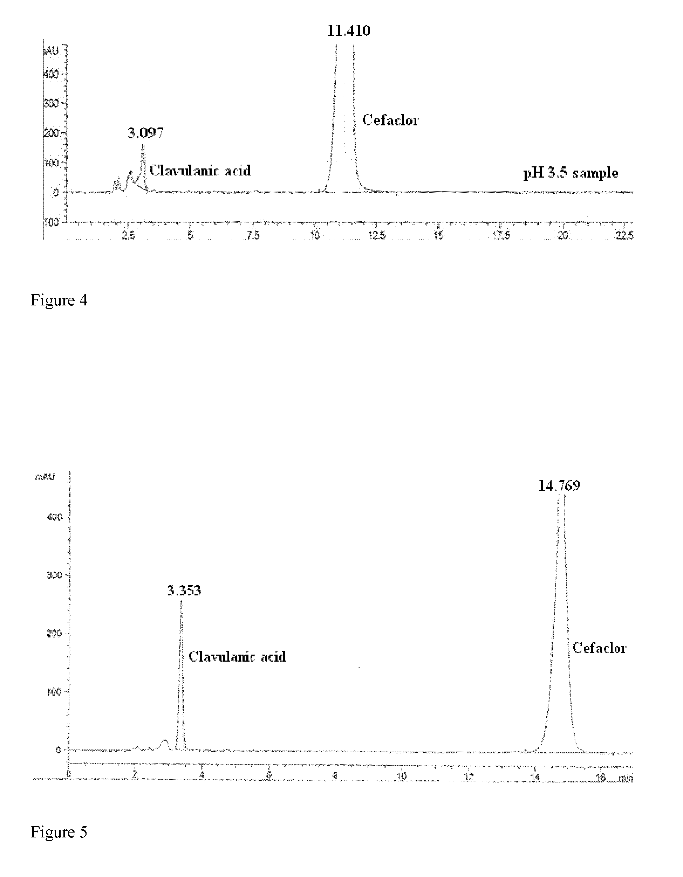

How the pH of the mobile phase effects the system has been examined. The prepared pH of the mobile phase has been adjusted to 3.5 as described above. Other method parameters have been fixed and the chromatograms have been recorded (Figure 3, figure 4). When the pH of the mobile phase was increased it was observed that the figures deteriorated and that clavulanic acid combined with the placebo peak. As the operation range of the column is pH 2.0 - 7.5, pH decreasing was not carried out. Under these conditions it has been decided for the pH to stay at 2.5 approximately.

### Determining the mobile phase organic adjuster amount

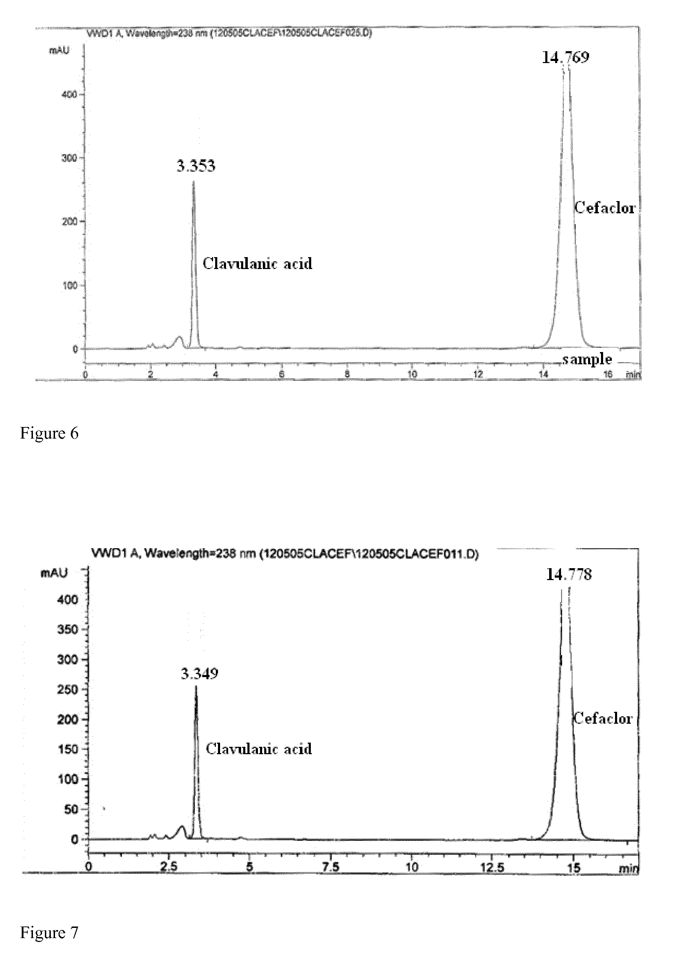

Sodium pentane sulfonate salt (1.0 g) is dissolved with 780 mL water. Triethylamin (10 mL) and methanol (350 mL) is added and mixed. Following this pH is is adjusted to 2.5 with ortho phosphoric acid. It is then drained through a membrane filter (0.45 µm), kept in an ultrasonic bath for 15 minutes and degassed.
Other conditions remained constant and by the increasing of methanol amounts, it has been enabled to observe peaks at an earlier time. However clavulanic acid peak has combined with small peaks received from the solvent. For this reason the methanol amount was decreased a little and a new mobile phase was prepared to carry out a trial.
Sodium pentane sulfonate salt (1.0 g) is dissolved with 780 mL water. Triethylamine (10 mL) and methanol (250 mL) is added and mixed. Following this pH is is adjusted to 2.5 with ortho phosphoric acid. It is then drained through a membrane filter (0.45 µm), kept in an ultrasonic bath for 15 minutes and degassed. The place and shape of the peaks have been optimized by means of the mobile phase prepared with these steps and chromatograms have been recorded (Figure 5, Figure 6).

### Physical characteristics of Cefaclor Clavulanic Acid Suspension (CLACEF)

Som physical tests have been applied to the Cefaclor Clavulanic Acid suspension formulation (CLACEF) trial. The powder which has been preapred has been diluted with mL water and shaken. After letting ti rest for a while, it has been topped off with water to obtain 100 mL. The test results have been given in Table 1.

**Table 1. Physical characteristics of Cefaclor Clavulanic Acid suspension (CLACEF)**

| **Tests applied** | **Result** |
|---|---|
| Suspendability | Very Good |
| pH | 4.74 |
| Intensity | 1.035 g/cm³ |
| Precitipation | Precitipation was not observed |

### Cefaclor Clavulanic acid amount determination method validation Selectivity

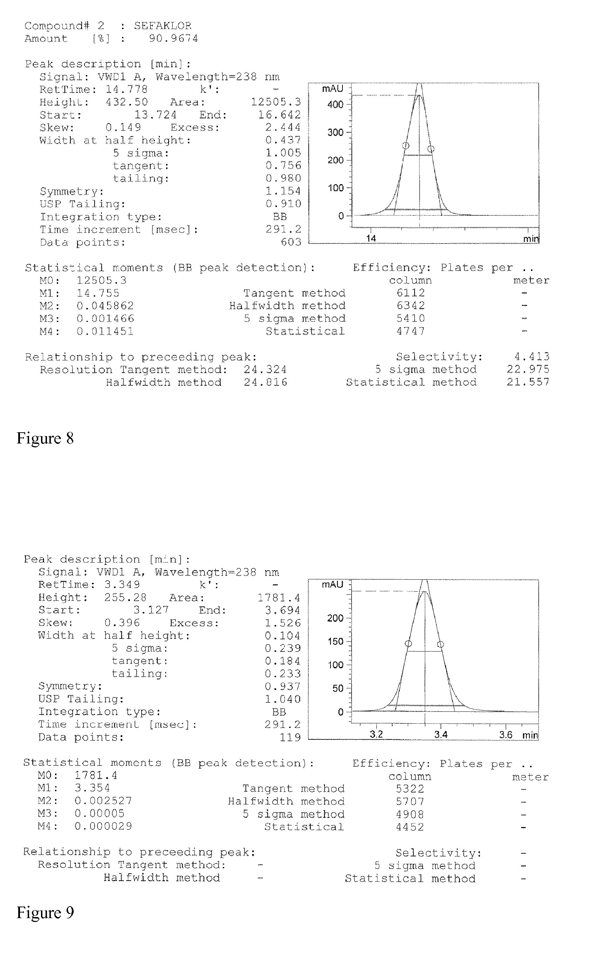

The system compatibility tests for the developed analysis method has been carried out by injecting YBSK-UV/GB to the detector system.
Following the injection of standard solvents to the system, the chomatographic parameters have been calculated by the device (Figure 7, Figure 8).
- The retention times of the peaks received from the placebo and solvent injections and the retention times of the active ingredients do not coincide.
- The cefaclor tailing factor has been recorded as 0.910 < 1.50, whereas the clavulanic acid tailing factor has been recoded as 0.937 < 1.50.
- The cefaclot theoretical plate number has been recorded as 6342 and the clavulanic acid theoretical plate number has been recorded as 5707.

### Calibration curve and linearity

Standard solutions having different concentrations between 0.24 mgml and 0.84 mg/ml for clavulanic acid, 0.5 mg/ml to 1.75mg for cefaclor have been prepared in order to determine the linear range and the calibration curve in the analysis method with high pressure liquid chromatography (HPLC) for cefaclor and clavulanic acid, and said solutions have been injected to high pressure liquid chromatography (HPLC). The test results have been given in Table 2 and Table 3.

**Table 2. Calibration curve characteristics belonging to the analysis method with high pressure liquid chromatography (HPLC)-UV/GB Detector for Clavulanic Acid**

| | |
|---|---|
| Correlation coefficient (r) | 0.99702 |
| Determination coefficient (r2) | 0.99405 |
| Curve (m) | 2335.95203 |
| Breakpoint of calibration line (b) | 259.88442 |

**Table 3. Calibration curve characteristics belonging to the analysis method with high pressure liquid chromatography (HPLC)-UV/GB Detector for Cefaclor**

| | |
|---|---|
| Correlation coefficient (r) | 0.99998 |
| Determination coefficient (r2) | 0.99996 |
| Curve (m) | 10088.05779 |
| Breakpoint of calibration line (b) | 67.81179 |

### Recovery

A synthetic mixture has been conducted by changing the concentration for the two active agents within the analysis method with HPLC-UV/GB for Cefaclor and Clavulanic acid. The concentrations prepared with an active agent and placebo are 80%, 100 % and 120 %. Samples having the composition 0.48 mg/mL, 0.6 mg/mL, 0.72 mg/mL for clavulanic acid and 1.0 mg/mL, 1.25 mg/mL ve 1.5 mg/mL for cefaclor have been prepared. The test results have been given in Table 4 and 5.

**Table 4. Clavulanic Acid recover % results**

| Theoretical concentration | Test concentration | % Recovery | % Approx. recorvery |
|---|---|---|---|
| 0.45 | 0.44 | 97.78 | 97.78 |
| 0.43 | 0.42 | 97.67 | |
| 0.42 | 0.41 | 97.62 | |
| 0.63 | 0.62 | 98.41 | 98.41 |
| 0.62 | 0.61 | 98,39 | |
| 0.62 | 0.61 | 98.39 | |
| 0.73 | 0.72 | 98.63 | 98.63 |
| 0.77 | 0.76 | 98.70 | |
| 0.78 | 0.76 | 97.44 | |

**Table 5. Cefaclor % Recovery results**

| Theoretical concentration | Test concentration | % Recovery | % Approx. recorvery |
|---|---|---|---|
| 0.94 | 0.95 | 101.06 | |
| 0.94 | 0.95 | 101.06 | |
| 0.94 | 0.95 | 101.06 | 101.06 |
| 1.21 | 1.2 | 99.17 | |
| 1.21 | 1.2 | 99.17 | |
| 1.21 | 1.19 | 98.35 | 99.17 |
| 1.49 | 1.48 | 99.33 | |
| 1.5 | 1.49 | 99.33 | |
| 1.51 | 1.49 | 98.68 | 99.33 |

### Method precision

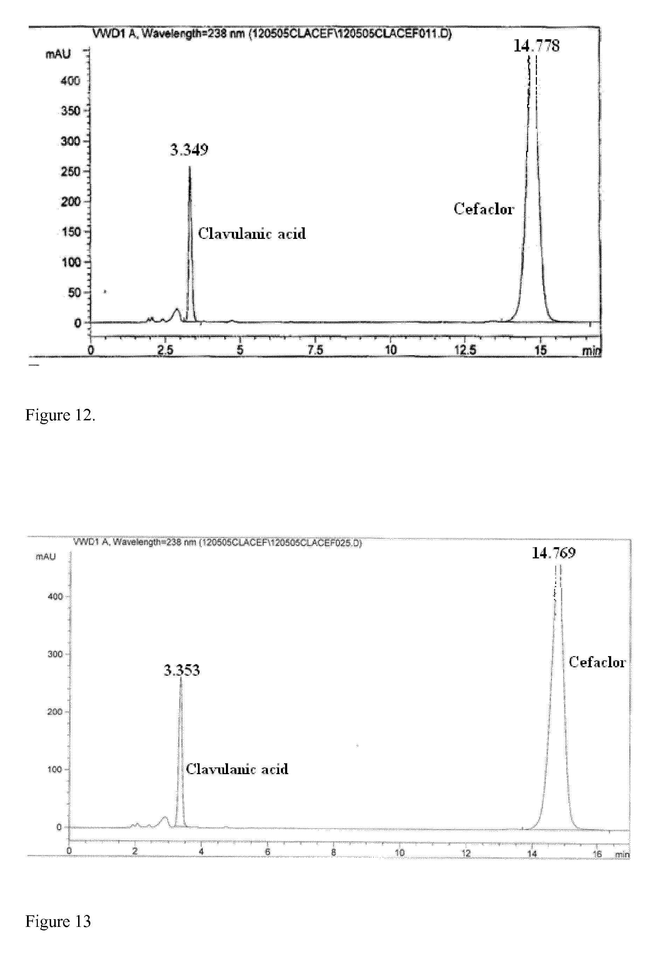

6 independent test solutions have been prepared for the precision study in order to determin the amount of Cefaclor and Clavulanic Acid. The clavulanic concentration in the test solutions prepared as mentioned above have a concentration of 0.60 mg/ml, whereas Cefaclor has a concentration of 1.25 mg/mL. The test solution chromatogram is shown in Figure 13. The method precision results are shown in Table 6 and 7.

**Table 6. Clavulanic Acid method precision results**

| **Sample Number** | **Clavulanic Acid %Amount** | **Approx** | **SS** | **BSS %** |
|---|---|---|---|---|
| 1 | 101.79 | 101.29 | 0.53 | 0.53 |
| 2 | 101.37 | | | |
| 3 | 100.95 | | | |
| 4 | 100.46 | | | |
| 5 | 101.27 | | | |
| 6 | 101.88 | | | |

**Table 7. Cefaclor Method precision results**

| **Sample Number** | **Cefaclor % Amount** | **Approx.** | **SS** | **BSS %** |
|---|---|---|---|---|
| 1 | 102.20 | 101.58 | 0.44 | 0.43 |
| 2 | 101.83 | | | |
| 3 | 101.79 | | | |
| 4 | 101.00 | | | |
| 5 | 101.34 | | | |
| 6 | 101.30 | | | |

In this study, a combined suspension product (CLACEF) study has been carried out between clavulanic acid which is a ß lactamase enzyme inhibitor and Cefaclor which is one of the ß lactamase group antibiotics which do not have medicine interaction and an analytic method has been developed. The desired taste and scent has not been obtained in the first trial during the clacef trial studies carried out. It has been determined that the swelling is low in the suspension, the intensity is low and that it is not well suspended. At the same time, in the amount determination analysis carried out it has been observed that the percentage recovery of cefaclor was low. Under the light of these results it has been decided that the formulation was not suitable for a dose of 250 mg Cefaclor/5 mL dose.

A new trial study has been carried out by evaluating the trial results that were conducted. In order to provide the desired taste, sodium saccharin has been used which has a sweetening taste 4 - 5 times more than aspartame. By this means it has been determined that the bitter taste was covered better. ß lactame products have a scent that is specific. It has been ensured that the scent was better by using strawberry scent. In order to solve the low swelling or a bad suspension problem that were faced in the initial trial, the xanthan gum which is the suspending agent was increased twofold. By this means a product which swelled more and suspended better was obtained. The dose of Cefaclor was decreased by 50 % and as a result the dissolving problem of Cefaclor is eliminated and the recovery of Cefaclor was close to 100 %. As a result it has been decided that the suspension formula was acceptable as 125 mg Cefaclor-57 mg Clavulanic Acid/5 mL.
The system compatibility parameters for the developed method have been determined and under the light of the parameters that have been obtained it has been decided that the method was applicable. In order to prove the validity of the method developed the parameters notified in the sources for the necessary method validation tests have been chosen and the related validity criteria have been accepted. For this aim, in the validation studies, linearity, selectivity, precision and recovery parameters have been examined and statistical evaluations thereof have been carried out. It has been observed that both the materials were linear at the range determined by means of the linearity study. The analytical method has been applied to the pharmaceutical dosage form and it has been determined that it can be used for both active ingredients. Recovery studies have been carried out in order to examine the effects of the admixtures inside the suspension, on the analysis method. According to the results obtained it has been decided that the suspension admixtures did not affect the analysis.
The formulation study conducted under the light of all this data suggests that the high pressure liquid chromatography (HPLC) method developed for the suspension formulation is recommended to determine the pharmaceutical dosage forms of Cefaclor and Clavulanic acid.

## Claims

1. A suspension composition comprising ceflactor and clavulanic acid to be administered orally.

2. A suspension to be administered orally wherein the cefaclor amount within 5 ml of the suspension is 12 5mg, and the clavulanic acid amount is 57 mg.

3. A composition according to claim 1 and 2, **characterized in that** it comprises a sweetener agent.

4. A composition according to claim 3, **characterized in that** the sweetener agent is sodium saccharin.

5. A composition according to claim 1 and 2, **characterized in that** it comprises a suspension agent.

6. A composition according to claim 4, **characterized in that** xanthan gum is used as a suspension agent.

7. A composition according to the preceding claims, **characterized in that**; the cefaclor and clavulanic acid analysis is carried out in high pressure liquid chromatography.

8. A high pressure liquid chromatography for carrying out cefaclor and clavulanic acid composition analysis, **characterized in that** the mobile phase used comprises sodium pentane sulfonate salt, triethylamine and methanol.

9. A mobile phase according to claim 8, **characterized in that**; the pH is adjusted with orthophosphoric acid.

10. A mobile phase according to claims 8 and 9, **characterized in that** the pH is adjusted to approximately 2.5.

11. A mobile phase according to claims 8 to 10 **characterized in that**; the mobile phase is drained through a 0.45 µm membrane filter before being applied to high pressure liquid chromatography.
